(19) Europäisches Patentamt
European Patent Office
Office européen des brevets

(11) Veröffentlichungsnummer: **0 173 142**
**B1**

(12) # EUROPÄISCHE PATENTSCHRIFT

(45) Veröffentlichungstag der Patentschrift: **31.05.89**

(21) Anmeldenummer: **85109979.6**

(22) Anmeldetag: **08.08.85**

(51) Int. Cl.⁴: **C 07 C 59/64,** C 07 C 59/80, C 07 C 59/84, C 07 C 33/26, C 07 C 69/736, C 07 C 103/34, C 07 D 307/32

(54) **Verfahren zur Herstellung von optisch aktiven Hydrochinonderivaten.**

(30) Priorität: **28.08.84 CH 4117/84**
**11.07.85 CH 3000/85**

(43) Veröffentlichungstag der Anmeldung: **05.03.86 Patentblatt 86/10**

(45) Bekanntmachung des Hinweises auf die Patenterteilung: **31.05.89 Patentblatt 89/22**

(84) Benannte Vertragsstaaten: **AT BE CH DE FR GB IT LI NL**

(56) Entgegenhaltungen:
**EP-A-0 107 806**
**EP-A-0 129 252**

**HELVETICA CHIMICA ACTA, Band 63, Nr. 8, 1980, Seiten 2451-2454, Verlag-Editions-Edizioni, Basel, CH; B. WEIDMANN et al.: "Methyl-triisopropoxy-titanium, a highly selective nucleophilic methylating reagent. Preliminary Communication"**
**ANGEWANDTE CHEMIE, Band 94, Nr. 2, 1982, Seiten 133-134, Weinheim; M.T. REETZ et al.: "Stereoselektivität und relative Reaktivität bei der Reaktion von Organotitan -und -zirkonium-Agentien mit Carbonylverbindungen"**

(73) Patentinhaber: **F. HOFFMANN- LA ROCHE & CO. Aktiengesellschaft, CH- 4002 Basel (CH)**

(72) Erfinder: **Richard, Barner, Dr., Traubenweg 16, CH-4108 Witterswil (CH)**
Erfinder: **Josef, Hübscher, Spausel 1080, CH- 5703 Seon (CH)**

(74) Vertreter: **Cottong, Norbert A., Grenzacherstrasse 124 Postfach 3255, CH- 4002 Basel (CH)**

**EP 0 173 142 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft ein neues Verfahren zur Herstellung von neuen Hydrochinonderivaten, welche als Zwischenprodukte für die Herstellung von d-α-Tocopherol (natürliches Vitamin E) geeignet sind. Die Erfindung betrifft ferner neue Ausgangsprodukte in diesem Verfahren.

Es sind bereits einige Verfahren zur Herstellung von natürlichem Vitamin E bekannt, welche jedoch technisch nur von begrenztem Interesse sind, so dass natürliches Vitamin E bis heute nahezu ausschliesslich aus natürlichen Quellen extrahiert wird. Es bestand somit ein Bedürfnis nach einem technisch realisierbaren Zugang, gemäss welchem natürliches Vitamin E in guter Ausbeute und mit hoher optischer Reinheit erhalten werden kann.

Das erfindungsgemässe Verfahren ist dadurch gekennzeichnet, dass man

a) eine Verbindung der allgemeinen Formel

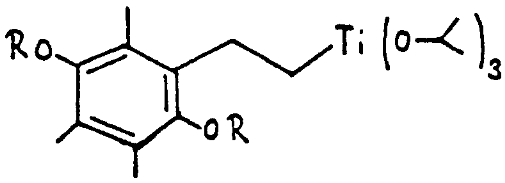

I

worin R eine Ätherschutzgruppe darstellt, mit einem Ketoester der Formel

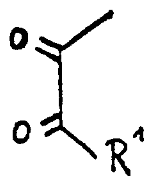

II

worin $R^1$ einen Rest der Formel

(a) 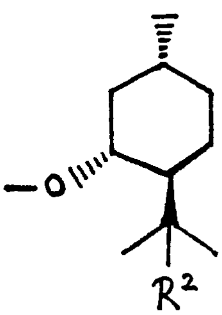

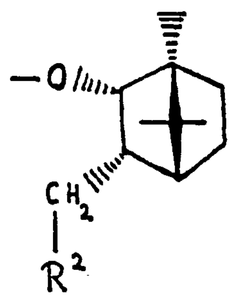 (b)

oder

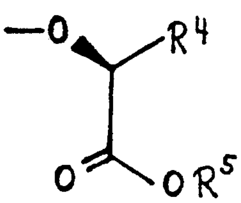

(c)

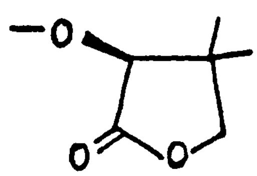

(d)

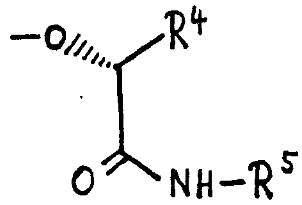

(e)

darstellt, $R^2$ die Phenylgruppe, $R^4$ Methyl oder Phenyl und $R^5$ niederes Alkyl, Aryl oder Aryl-niederes Alkyl bedeuten,

umsetzt und dass man, gewünschtenfalls, eine so erhaltene Verbindung der allgemeinen Formel

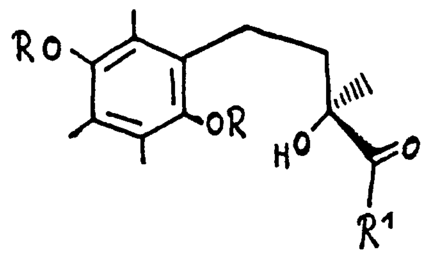

III

worin R und $R^1$ obige Bedeutung haben,

zu einer Verbindung der allgemeinen Formel

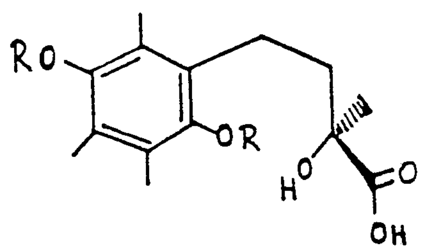

IV

worin R die obigen Bedeutung hat,

hydrolysiert, oder zu einer Verbindung der allgemeinen Formel

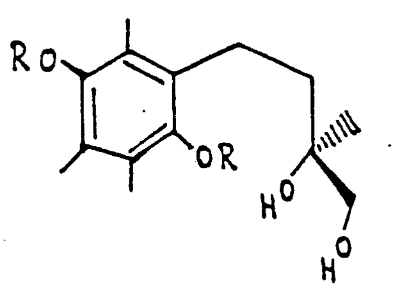

V

worin R obige Bedeutung haben,

reduziert, oder dass man

b) die Verbindung der Formel

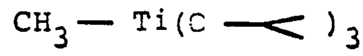

VI

mit einer Verbindung der allgemeinen Formel

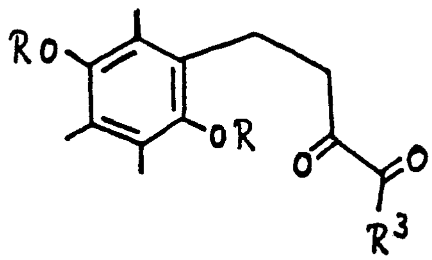

VII

worin $R^3$ einen Rest der Formel

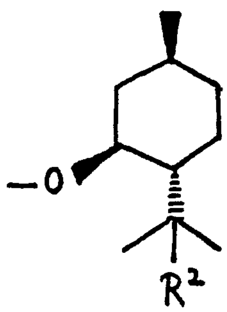

(f)

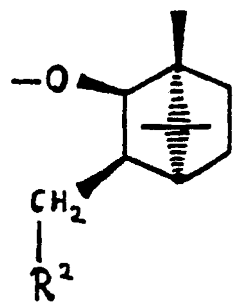

(g)

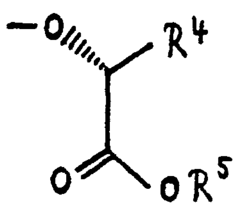

(h)

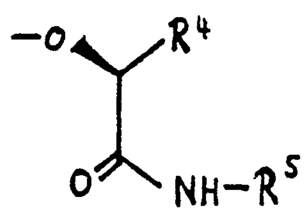

(i)

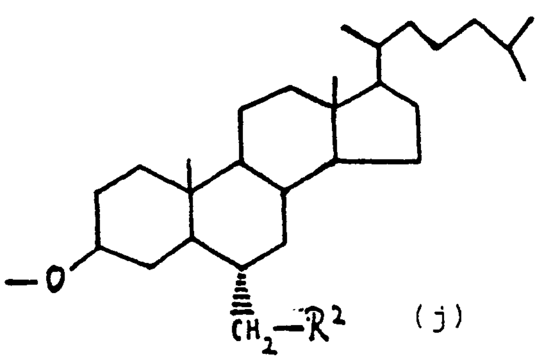

(j)

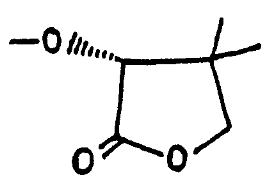

(k)

darstellt und R, $R^2$, $R^4$ und $R^5$ die obige Bedeutung haben,

umsetzt und dass man, gewünschtenfalls, eine so erhaltene Verbindung der allgemeinen Formel

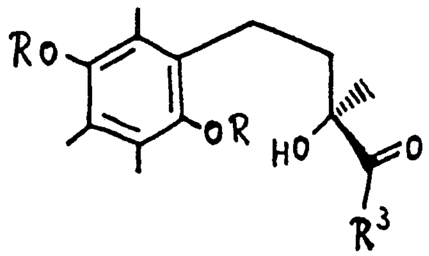

III-A

worin R und $R^3$ die obigen Bedeutungen haben, zu einer Verbindung der allgemeinen Formel IV hydrolysiert oder zu einer Verbindung der allgemeinen Formel V reduziert.

Der Ausdruck "Ätherschutzgruppe" bedeutet im Rahmen der vorliegenden Erfindung sowohl durch Hydrolyse spaltbare Gruppen wie etwa die Silylgruppe oder Alkoxymethylgruppen, beispielsweise die Methoxymethylgruppe, oder auch die Tetrahydropyranylgruppe, als auch oxidativ spaltbare Gruppen wie etwa $C_1$-$C_6$ Alkyläthergruppen

Der Ausdruck "niederes Alkyl" bedeutet im Rahmen der vorliegenden Erfindung geradkettige oder verzweigte Alkylgruppen mit 1 - 6 Kohlenstoffatomen, wie Methyl, Äthyl, Propyl, Isopropyl, n-Butyl, Isobutyl, tert. Butyl, Pentyl, Hexyl und dergleichen. Der Ausdruck "Aryl" bedeutet sowohl Phenyl als auch Naphthyl welche sowohl substituiert als auch unsubstituiert sein können. In dem Ausdruck "Aryl-niederes Alkyl" haben Aryl und niederes Alkyl die vorhergehende Bedeutung.

Weiterhin bedeutet das Zeichen "FIGFIG", dass sich der entsprechende Rest oberhalb der Molekülebene befindet, während das Zeichen "FIGFIG" bedeutet, dass sich der entsprechende Rest unterhalb der Molekülebene befindet.

Die Verbindungen der Formeln I, III, IIIA, IV und VII sind neu und ebenfalls Gegenstand der vorliegenden Erfindung.

Die Umsetzung einer Verbindung der Formel I mit dem Ketoester der Formel II erfolgt zweckmässig in einem inerten organischen Lösungsmittel bei einer Temperatur von etwa -80°C bis etwa +10°C, vorzugsweise bei etwa -20°C bis etwa 0°C. Als Lösungsmittel können hier insbesondere diejenigen genannt werden, welche üblicherweise bei metallorganischen Reaktionen verwendet werden, insbesondere Äther wie etwa Diäthyläther, tert. Butylmethyläther, Tetrahydrofuran und dergleichen.

Die Hydrolyse einer Verbindung der Formel III zu einer Hydroxysäure der allgemeinen Formel IV kann in an sich bekannter Weise erfolgen. Zweckmässig erfolgt diese Hydrolyse in einem geeigneten organischen Lösungsmittel wie etwa einem niederen Alkohol mit 1 - 5 Kohlenstoffatomen, z. B. Methanol, Äthanol und dergleichen, mittels eines Alkali- oder Erdalkalihydroxides, vorzugsweise mittels Natrium- oder Kaliumhydroxid.

Die Reduktion einer Verbindung der Formel III zu einer Verbindung der Formel V kann in an sich bekannter Weise durchgeführt werden. Zweckmässig erfolgt dies mittels komplexer Metallhydride wie z. B. $LiAlH_4$, $LiBH_4$, Diisobutylaluminiumhydrid und dergleichen. Als Lösungsmittel kommen inerte organische Lösungsmittel in Frage, insbesondere Äther wie Diäthyläther oder Tetrahydrofuran und dergleichen. Die Temperatur und der Druck sind keine kritischen Grössen in dieser Reaktion, so dass diese unter den bei Reduktionen von Estern zu Alkoholen üblichen Bedingungen durchgeführt werden kann.

Die als Ausgangsmaterial verwendeten Ketoester der Formel II sind bekannte Verbindungen oder Analoge bekannter Verbindungen, welche leicht in zur Herstellung der bekannten Verbindungen analoger Weise hergestellt werden können. Die ebenfalls als Ausgangsmaterial verwendeten Verbindungen der Formel I sind jedoch neu und können gemäss folgendem Beaktionsschema hergestellt werden.

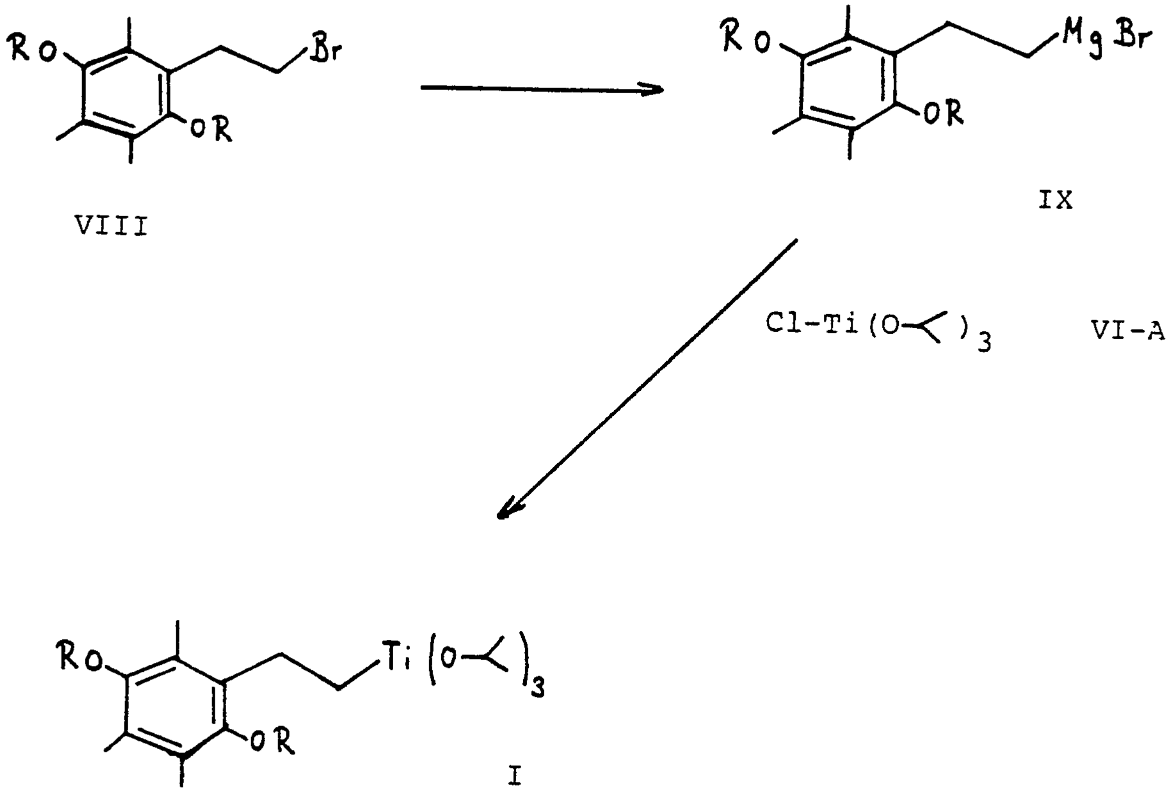


VIII

IX

I

worin R die obige Bedeutung hat.

Die Überführung der Verbindungen der Formel VIII in die Grignardverbindungen der Formel IX kann unter den zur Herstellung von Grignardverbindungen üblichen Bedingungen erfolgen.

Die Umsetzung einer Grignardverbindung der Formel IX mit der Verbindung der Formel VI - A kann in an sich bekannter Weise durchgeführt werden. Zweckmässig erfolgt dies bei etwa -80°C bis etwa 0°C, vorzugsweise bei -20°C. Als Lösungsmittel können alle diejenigen verwendet werden, welche auch bei der Herstellung der Grignardverbindungen in Betracht kommen.

Die Umsetzung der Verbindung der Formel VI mit einer Verbindung der Formel VII kann in an sich bekannter Weise, insbesondere in zur Umsetzung einer Verbindung der Formel I mit einem Ketoester der Formel II analoger Weise durchgeführt werden.

Die als Ausgangsmaterial verwendete Verbindung der Formel VI ist bekannt. Die ebenfalls als Ausgangsmaterial verwendeten Verbindungen der Formel VII sind jedoch neu und ebenfalls Gegenstand der vorliegenden Erfindung. Sie können wie im folgenden Schema dargestellt hergestellt werden.

Schema

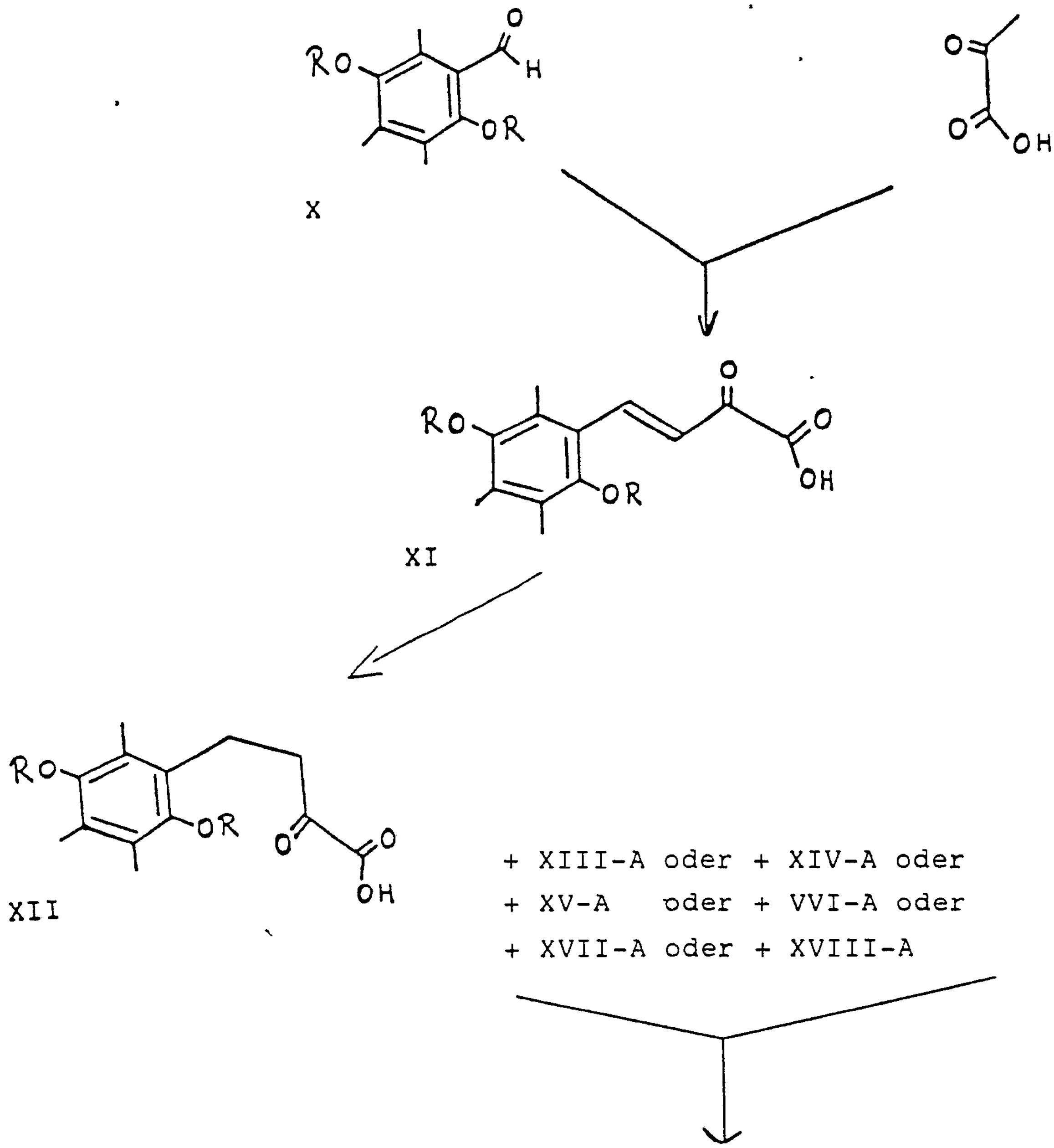

X

XI

XII

VII

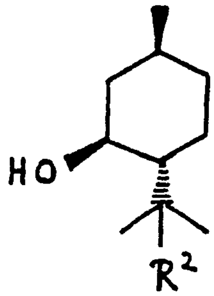

XIII-A

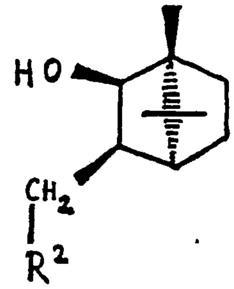

XIV-A

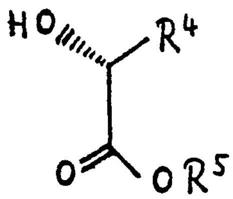

XV-A

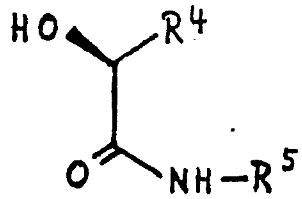

XVI-A

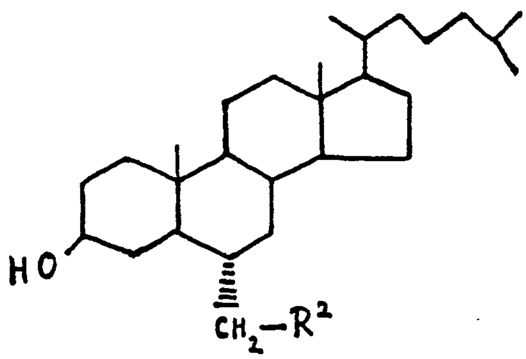

XVII-A

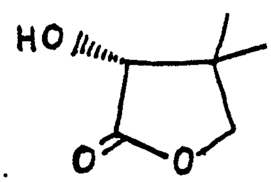

XVIII-A

worin R, $R^2$, $R^4$ und $R^5$ die obigen Bedeutungen haben.

Die Umsetzung einer Verbindung der Formel X mit der Brenztraubensäure kann in an sich bekannter Weise erfolgen. Zweckmässig erfolgt dies in wässrig-alkoholischem Medium unter Zusatz einer Base wie etwa Kalium- oder Natriumhydroxid. Die Reduktion einer Verbindung der Formel XI zu einer Verbindung der Formel XII kann ebenfalls in an sich bekannter Weise, vorzugsweise katalytisch in Gegenwart einer Base erfolgen. Als Katalysator kann insbesondere Palladium verwendet werden. Als Basen sind anorganische Basen wie Natrium- oder Kaliumhydroxid oder auch organische Basen wie Triäthylamin und dergleichen verwendbar.

Die Umsetzung einer Verbindung der Formel XII mit einer Verbindung der Formeln XIII - A bis XVIII - A kann in zur Herstellung von Estern aus Säuren und Alkoholen üblicher Weise erfolgen. Vorzugsweise erfolgt die Veresterung, indem die Säure zunächst in das Imidazolid übergeführt wird und dieses dann mit dem Alkohol umgesetzt wird.

Die erfindungsgemäss hergestellten Verbindungen der Formeln IV und V sind wertvolle Zwischenprodukte in der Synthese von natürlichem Vitamin E.

Die Verbindungen der Formel IV können beispielsweise in die bekannte Verbindung der allgemeinen Formel

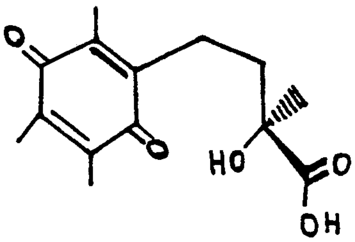

XIX

übergeführt werden und diese in bekannter Weise in natürliches Vitamin E. Diese Überführung der Verbindungen der Formel IV in diejenigen der Formel XIX kann in an sich bekannter Weise erfolgen. Zweckmässig erfolgt dies durch Oxidation mittels $Ce(NH_4)_2(NO_3)_6$ in wässrigem Acetonitril.

Die Verbindungen der Formel V können beispielsweise dadurch in natürliches Vitamin E übergeführt werden, dass man die Verbindungen der Formel V in Epoxide überführt, die erhaltenen Epoxide der allgemeinen Formel

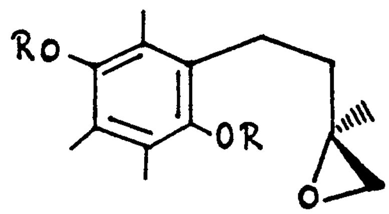

XX

worin R die obige Bedeutung hat,

mit der Grignard-Verbindung der allgemeinen Formel

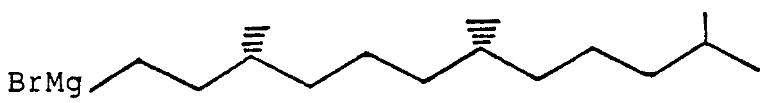

XXI

umsetzt und so erhaltene bekannte Verbindungen der allgemeinen Formel

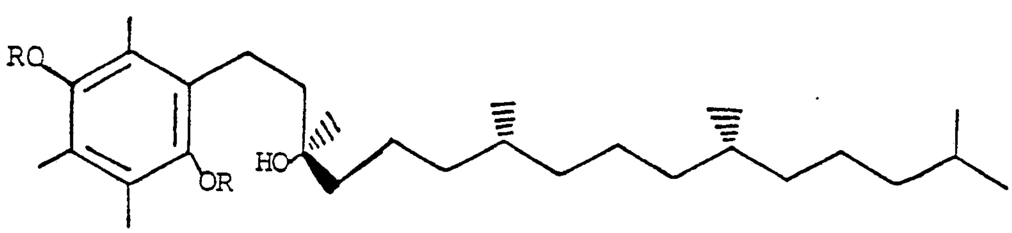

XXII

worin R die obige Bedeutung hat,

in bekannter Weise in d-α-Tocopherol überführt.

Die Überführung einer Verbindung der Formel V in ein Epoxid der Formel XX kann in an sich bekannter Weise durchgeführt werden. Zu diesem Zweck wird in einer Verbindung der Formel V zunächst die primäre Hydroxygruppe in eine Abgangsgruppe übergeführt, z. B. in ein Halogenid (als Halogen kommen hier Chlor, Brom und Jod in Frage) oder in einen Sulfonsäureester (z. B. Tosylat oder Mesylat) und dergleichen. Dies kann in an sich bekannter Weise erfolgen. Anschliessend wird die so erhaltene Verbindung mit einer Base behandelt. Als Basen eignen sich sowohl anorganische als auch organische Basen, vorzugsweise jedoch anorganische Basen wie insbesondere Natriumhydroxid oder Kaliumhydroxid und dergleichen.

Die Umsetzung eines Epoxids der Formel XX mit der Grignard-Verbindung der Formel XXI kann in an sich bekannter Weise durchgeführt werden. Bevorzugt ist jedoch die Umsetzung in Gegenwart von Kupfer (I oder II) -Katalysatoren, insbesondere Kupfer (I)-n-Propylacetylid oder einem Kupfer (I)-halogeniddimethylsulfid-Komplex. Als Lösungsmittel eignen sich für diese Umsetzung alle bei Grignard-Reaktionen üblicherweise in Frage kommenden Lösungsmittel.

Die Verbindungen der allgemeinen Formel XXII sind bekannt und können in bekannter Weise in d-α-Tocopherol übergeführt werden.

**Beispiel 1**

Eine Lösung von 18,6 mMol 2-(2',5'-Dimethoxy-3',4',6',-trimethylphenyl)äthyl triisopropoxytitan in 20 ml Tetrahydrofuran wird bei -20°C tropfenweise mit 3,12 g (10,3 mMol) Brenztraubensäure-(-)-8-phenylmethylester unter Rühren versetzt und das Gemisch anschliessend noch 16 Stunden bei -20°C stehen gelassen. Nach der Zugabe von 50 ml wässriger Natriumdihydrogenphosphatlösung (10 %-ig) wird 3 Mal mit je 50 ml Äther extrahiert. Die vereinigten organischen Phasen werden über Natriumsulfat getrocknet und dann das Lösungsmittel am Rotationsverdampfer entfernt. Es werden 6,76 g (S)-4-(2',5'-Dimethoxy-3',4',6'-trimethylphenyl)-2-hydroxy-2-methyl-butansäure-(-)-8-phenylmenthylester erhalten.

Das als Ausgangsmaterial verwendete 2-(2'5'-Dimethoxy-3',4',6'-trimethylphenyl)-äthyl triisopropoxytitan kann wie folgt hergestellt werden:

5,34 g (18,6 mMol) 1-(2-Bromäthyl)-2,5-dimethoxy-3,4,6-trimethylbenzol werden in 20 ml trockenem Tetrahydrofuran mit 0,50 g (20,8 mMol) Magnesium während 1 Stunde am Rückfluss erhitzt. Dann werden bei -20°C 4,71 ml (19,0 mMol) Chlor-triisopropoxy-titan zugegeben. Die Lösung des entstandenen 2-(2',5'-Dimethoxy-3',4',6'-trimethylphenyl)-äthyl triisopropoxytitan wird direkt weiterverarbeitet.

**Beispiel 2**

Eine Lösung von 0,907 g (ca 1,35 mMol) (S)-4-(2',5'-Dimethoxy-3',4',6'-trimethylphenyl)-2-hydroxy-2-methylbutansäure-(-)-8-phenylmenthylester in 10 ml Äthanol wird mit 1 ml 28 %-iger Natronlauge versetzt und das Gemisch während 16 Stunden bei Raumtemperatur stehen gelassen. Dann wird mit 50 ml Wasser verdünnt, und dreimal mit je 50 ml Äther extrahiert. Die wässrige Phase wird mit 10 %-iger Phosphorsäure angesäuert (pH 2) und dreimal mit je 50 ml Äther extrahiert. Die vereinigten organischen Phasen werden über Natriumsulfat getrocknet und das Lösungsmittel anschliessend am Rotationsverdampfer entfernt. Man erhält 212 mg (S)-4-(2',5'-Dimethoxy-3',4',6'trimethylphenyl)-2-hydroxy-2-methylbutansäure.

**Beispiel 3**

Eine Lösung von 6,76 g (ca. 10,3 mMol) (S)-4-(2',5'-Dimethoxy-3',4',6'-trimethylphenyl)-2-hydroxy-2-methylbutansäure-(-)-8-phenylmenthylester in 50 ml trockenem Äther wird unter Rühren zu einer Suspension von 0,380 g (9,98 mMol) $LiAlH_4$ in 10 ml Äther zugetropft. Nach weiterem zweistündigem Rühren bei Raumtemperatur werden 10 ml Äthylacetat zugetropft (Zerstörung von überschüssigem $LiAlH_4$) und anschliessend 10 ml 10 %-ige wässrige Natriumdihydrogenphosphatlösung zugetropft. Das Reaktionsgemisch wird 3 Mal mit je 50 ml Äther extrahiert, die vereinigten organischen Phasen über Natriumsulfat getrocknet und das Lösungsmittel am Rotationsverdampfer entfernt, wobei 4,61 g rohes (S)-4-(2',5'-Dimethoxy-3',4',6'-trimethylphenyl)-2-methyl-1,2-butandiol erhalten werden. Das Rohprodukt wird aus 10 ml Äther und 40 ml Hexan umgefällt und ergibt 1,68 g des reinen Diols als weisses Produkt.

Smp. 85 - 86°; $[\alpha]^{25}_D = +2,79°$ (c = 2 % in Chloroform).

Die Verbindung erweist sich als Acetonid im $^1H$-NMR (60 MHz) mit $Eu(HFC)_3$ als chiralem Shiftreagenz als optisch rein (e.e ) > 90 %).

**Beispiel 4**

Eine Lösung von 0.78 Mol 2-(2',5'-Dimethoxy-3',4',6'-trimethylphenyl)-äthyltriisopropoxytitan (hergestellt gemäss Beispiel 1) in 10 ml trockenem Tetrahydrofuran wird bei -20°C mit 0.30 g (0.95 Mol) Brenztraubensäure-(+)-cis-3-benzylbornylester unter Rühren versetzt und das Gemisch anschliessend noch 16 Stunden bei -20°C stehen gelassen. Die Aufarbeitung erfolgt in zu Beispiel 1 analoger Weise und man erhält (S)-4-(2',4'-Dimethoxy-3',4',6'-trimethylphenyl)-2-hydroxy -2-methyl-butansäure-(+)-cis-3-benzylbornylester, welcher direkt in zu Beispiel 2 analoger Weise verseift wird. Die dabei erhaltene wässrige Phase wird mit 0.5 ml Dimethylsulfat und 20 mg Aliquat 336 (ein Phasentransferkatalysator von der Firma Fluka/CH) versetzt und 1 Stunde bei Raumtemperatur gerührt. Anschliessend hat man 1 ml 25 %-igen Ammoniak zugefügt und noch 1 Stunde bei Raumtemperatur gerührt, (Entfernung von überschüssigem Dimethylsulfat). Durch Extraktion des Reaktionsgemisches mit Äther, Trocknen der Ätherphase über Natriumsulfat und Entfernen des Lösungsmittels erhält man 150 mg (S)-4-(2',5'-Dimethoxy-3',4',6'-trimethylphenyl)-2-hydroxy-2-methylbutansäure-methylester. Optische Reinheit gemäss HPLC an einer "Pirkle-Phase" = 95 % e.e.

Der als Ausgangsmaterial verwendete Brenztrauben-säure-(+)-cis-3-benzylbornylester kann wie folgt hergestellt werden:

Eine Lösung von 0.88 g (10 mMol) Brenztraubensäure in 20 ml trockenem Methylenchlorid werden mit 2.2 g (10 mMol) Carbonylimidazol versetzt und das Gemisch bis zur Beendigung der Gasentwicklung gerührt (~ 10

Minuten). Zu der so erhaltenen Lösung von Brenztraubensäureimidazolid werden 3,7 g (10 mMol) (+)-cis-3-Benzylborneol in trockenem Methylenchlorid zugefügt zusammen mit 1 mMol Imidazollithium als Katalysator. Das Gemisch wird über Nacht bei Raumtemperatur gerührt, anschliessend mit Wasser und Äther aufgenommen, die Ätherphase durch Kieselgel filtriert, eingeengt und der Rückstand im Hochvakuum ($10^{-2}$Torr) bei ~ 190°C destilliert. Man erhält 4,5 g Brenztraubensäure-(+)-cis-3-benzyl-bornylester. Dünnschichtchromatographie: (Kieselgel/Toluol-Essigester 10 : 1): Rf = 0,4.

**Beispiel 5**

Eine Lösung von 1,4 g (2,68 mMol) 4(2',5'-Dimethoxy-3',4',6'-trimethylphenyl)-2-keto-butansäure-(-)-cis-3-benzylbornylester in 20 ml trockenem Tetrahydrofuran wird unter Rühren bei -20°C mit 3 mMol Methyltriisopropoxytitan in 1 ml Hexan versetzt und dieses Gemisch anschliessend noch 16 Stunden bei -20°C stehen gelassen. Die Aufarbeitung erfolgt in zu Beispiel 1 analoger Weise und man erhält S-4-(2',5'-Dimethoxy-3',4',6'-trimethylphenyl)-2-hydroxy-2-methylbutansäure(-)-cis-3-benzylbornylester, welcher in zu Beispiel 4 analoger Weise in den Methylester übergeführt wird.

Optische Reinheit gemäss HPLC an einer "Pirkle-Phase": 70 % e.e. Die optische Reinheit des ursprünglich verwendeten (-)-Borneol betrug ebenfalls 70 % e.e.

Der in zum Vorhergehenden analoger Weise mit optisch reinem (+)-Borneol durchgeführte Versuch ergab den entsprechenden (R)-Methylester mit einer optischen Reinheit von 95 % e. e.

Der als Ausgangsmaterial verwendete 4-(2',5'-Dimethoxy-3',4',6'-trimethylphenyl)-2-keto-butansäure-(-)-cis-3-benzylbornylester kann in zu Beispiel 4 analoger Weise ausgehend von 4-(2',5'-Dimethoxy-3',4',6'-trimethylphenyl)-2-ketobutansäure und (-)-cis-3-Benzylborneol hergestellt werden.

Die hierzu wiederum als Ausgangsmaterial verwendete 4-(2',5'-Dimethoxy-3',4',6'-trimethylphenyl)-2-ketobutansäure kann wie folgt hergestellt werden:

Eine Suspension von 3,7 g (17,6 mMol) 2,5-Dimethoxy-3,4,6-trimethylbenzaldehyd und 5,0 g (53,2 mMol) Natriumpyruvat (Brenztraubensäure-Natriumsalz) in 20 ml Methanol wird mit 0.1 g (1.8 mMol) Kaliumhydroxyd versetzt und anschliessend während 5 Stunden am Rückfluss erhitzt, wobei eine gelbe Lösung erhalten wird. Das Reaktionsgemisch wird auf Eiswasser gegossen und durch Zugabe von Schwefelsäure unter Rühren die entstandene 4-(2',5'-Dimethoxy-3-,4',6'-trimethylphenyl)-2-keto-3-butensäure ausgefällt. Die Suspension wird während 1 Stunde gerührt, filtriert, der Filterrückstand mit Wasser gewaschen und getrocknet. Es werden auf diese Weise 4,9 g der 4-(2',5'-Dimethoxy-3',4',6'-trimethylphenyl)-2-keto-3-butensäure in Form eines gelbes Pulvers mit Smp. 126 - 127°C erhalten.

1,02 g (3,5 mMol) dieses Pulvers in 80 ml Wasser werden unter Zusatz von 10 ml 1N Natronlauge über 400 mg Pd/C (5 %) unter Normaldruck hydriert, bis die dünnschichtchromatographische Kontrolle vollständige Hydrierung anzeigt. Der Katalysator wird abfiltriert, das Filtrat mit Phosphorsäure auf pH 4 angesäuert und mit Äther extrahiert. Durch Eindampfen der über Natriumsulfat getrockneten Ätherextrakte werden 0,75 g 4-(2',5'-Dimethoxy-3',4',6'-trimethylphenyl)-2-keto-butansäure erhalten.

**Beispiel 6**

In zu Beispiel 5 analoger Weise wird 4-(2',5'-Di-methoxy-3',4',6'-trimethylphenyl)-2-keto-butansäure-(1S)-1-benzylcarbamyl-äthylester mit Methyl-triisopropoxytitan zuerst 5 Stunden bei -78°C und dann 16 Stunden bei -20°C umgesetzt und das Reaktionsprodukt in den Methylester übergeführt. Die optische Reinheit beträgt laut HPLC an einer "Pirkle-Phase" über 95 % e.e.

Der als Ausgangsmaterial verwendete (1S)-1-Benzylcarbamyl-äthylester kann in zu Beispiel 4 analoger Weise aus 4-(2',5'-Dimethoxy-3',4',6'-trimethylphenyl)-2-ketobutan-säure und (1S)-1-(Benzylcarbamyl)-äthanol (= L-Milchsäurebenzylamid) hergestellt werden.

**Beispiel 7**

148 mg (0,5 mMol) (S)-4-(2',5'-Dimethoxy-3',4',6'-trimethylphenyl)-2-hydroxy-2-methylbutansäure (hergestellt gemäss Beispiel 2) werden in 2 ml Acetonitril gelöst und unter Rühren mit 560 mg (1 mMol) $Ce(NH_4)2(NO_3)_6$ in 2 ml Wasser versetzt und das Gemisch wird anschliessend noch 10 Minuten bei Raumtemperatur gerührt. Dann wird mit 50 ml Wasser versetzt und 3 Mal mit je 50 ml Chloroform extrahiert. Die vereinigten organischen Phasen werden nach dem Trocknen über Natriumsulfat eingeengt und dabei werden 111 mg (+)-(S)-2-Hydroxy-2-methyl-4-(2',4',5'-trimethyl-3',6'-dioxo-1',4'-cyclohexadien-1'-yl)-butansäure erhalten.

Smp: 115 - 117°C; $[\alpha]^{25}_D = +11°$ (c = 0,2 in Chloroform); optische Reinheit des Eduktes gemäss HPLC an "Pirkle-Phase" (optische aktive Phase) 95 % e.e.

Das nach katalytischer Reduktion und anschliessender saurer Cyclisierung erhaltene Produkt, d.h. (-)-(S)-6-Hydroxy-2,5,7,9-tetramethylchroman-2-carbonsäure, wird nach Umkristallisation aus Toluol optisch rein erhalten. $[\alpha]^{25}_D$ = -68,8° (c = 1 % in Chloroform).

**Beispiel 8**

a) 237 mg Tosylchlorid und 350 mg (S)-4-(2',5',-Dimethoxy-3',4',6'-trimethylphenyl)-2-methyl-1,2-butandiol wurden in 1 ml Methylenchlorid gelöst. Dann wurden bei 0°C 0,180 ml Pyridin zugetropft und das Gemisch 1 Stunde bei 0°C und dann 16 Stunden bei Raumtemperatur stehen gelassen. Hierauf wurden 1 g Eis und 0,3 ml konzentrierte Salzsäure zugegeben. Dann wurde mit Methylenchlorid extrahiert, der Extrakt getrocknet und eingeengt. Man erhielt 511 mg (95 %) (S)-4-(2',5'-Dimethoxy-3',4',6'-trimethylphenyl)-2-methyl-1-toluylsulfonyloxy-2-butanol.

$[\alpha]^{20}_D$ +1,2° (c = 2,6 % in Chloroform).

b) 177 mg (S)-4-(2',5',-Dimethoxy-3',4',6'-trimethylphenyl)-2-methyl-1-toluylsulfonyloxy-2-butanol wurden in 1 ml Äthanol gelöst und mit 0,3 ml alkoholischer Kalilauge (1,5N) versetzt. Das Gemisch wurde 10 Minuten bei Raumtemperatur stehen gelassen, dann werden 30 ml Methylenchlorid zugegeben und das Gemisch über Natriumsulfat getrocknet und eingeengt. Man erhielt 105 mg (S)-4-(2',5'-Dimethoxy-3',4',6'-trimethylphenyl)-2-methyl-1,2-epoxybutan. Smp. 47 - 48°C.

$[\alpha]^{20}_D$ +4,91° (c = 2,2 % in Chloroform).

c) 5,8 mMol (3R, 7R)-3,7,11-Trimethyl-dodecylbromid wurden in 20 ml Äthyläther mit geätztem Magnesium 1/4 Stunde am Rückfluss erhitzt. Dann wurden bei 0°C 1 g (S)-4-(2',5'-Dimethoxy-3',4',6'-trimethylphenyl)-2-methyl-1,2-epoxybutan, 0,9 g Kupfer(I)-2-propylacetylid (bzw. 1,2 g Kupfer(I)-bromid-dimethylsulfid-Komplex) zugesetzt. Die Temperatur des Reaktionsgemisches wurde anschliessend auf Raumtemperatur ansteigen gelassen und das Gemisch wurde über Nacht gerührt. Dann wurden 10 ml Ammoniumchlorid zugegeben und das Gemisch mit Äthyläther extrahiert. Der Extrakt wurde getrocknet, eingeengt und im Kugelrohr destilliert ($Kp_{0,01}$ = 140°C). Man erhält 1,28 g (72 %) [bzw. 1,41 g (79 %)] ( 3R, 7R, 11R)-1-(2',5',-Dimethoxy-3',4',6'-trimethylphenyl)-3,7,11,15-tetramethyl-hexadecan-3-ol als farbloses Öl.

$[\alpha]^{20}_D$-0,67° (c = 0,9 % in Chloroform).

| | | |
|---|---|---|
| $C_{31}H_{56}O_3$ (476,79) | Ber.: | C = 78,09 H = 11,84 |
| | Gef.: | C = 77,92 H = 11,88 |

**Patentansprüche** für die Vertragsstaaten: BE, CH, DE, FR, GB, IT, LI, NL.

1. Verfahren zur Herstellung von Hydrochinonderivaten, dadurch gekennzeichnet, dass man

a) eine Verbindung der allgemeinen Formel

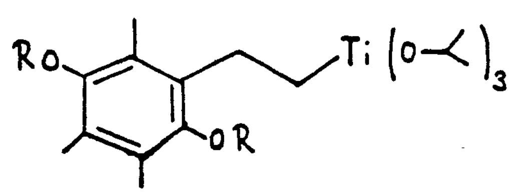

I

worin R eine Ätherschutzgruppe darstellt, mit einem Ketoester der Formel

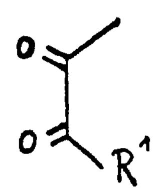

II

vor in $R^1$ einen Rest der Formel

(a) 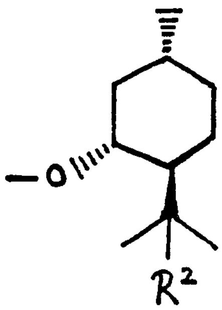

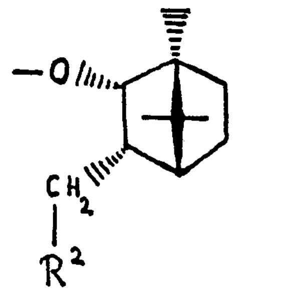 (b)

oder

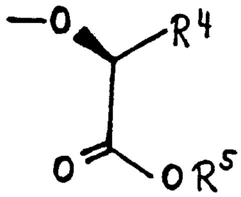

(c)

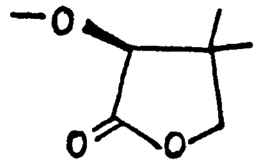

(d)

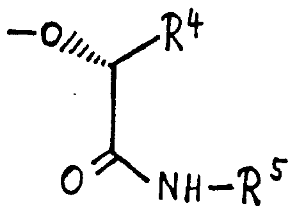

(e)

darstellt, $R^2$ die Phenylgruppe, $R^4$ Methyl oder Phenyl und $R^5$ niederes Alkyl, Aryl oder Aryl-niederes Alkyl bedeuten,

umsetzt und dass man, gewüschtenfalls, eine so erhaltene Verbindung der allgemeinen Formel

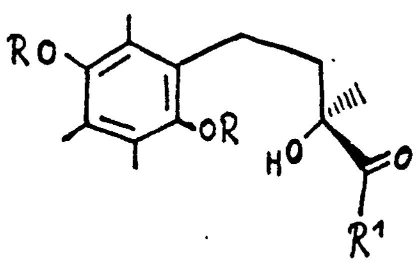

III

worin R und $R^1$ obige Bedeutung haben,

zu einer Verbindung der allgemeinen Formel

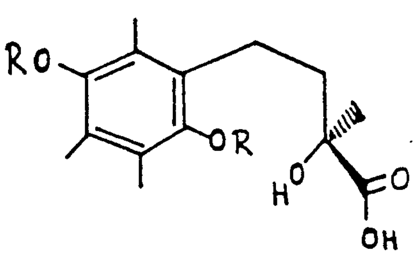

IV

worin R die obige Bedeutung hat,

hydrolysiert, oder zu einer Verbindung der allgemeinen Formel

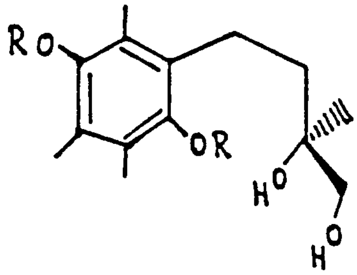

V

worin R obige Bedeutung hat,
reduziert, oder dass man
b) die Verbindung der Formel

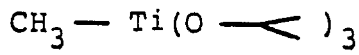

VI

mit einer Verbindung der allgemeinen Formel

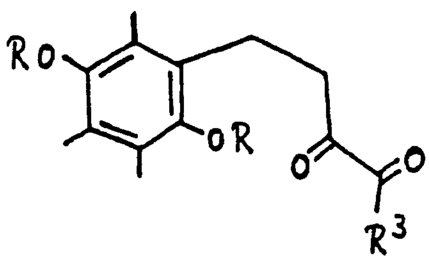

VII

worin $R^3$ einen Rest der Formel

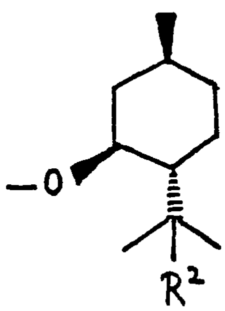

(f)

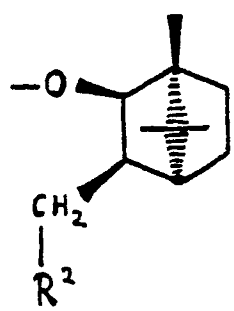

(g)

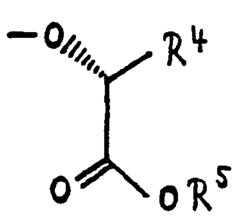

(h)

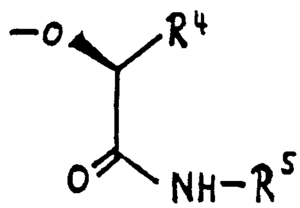

(i)

oder

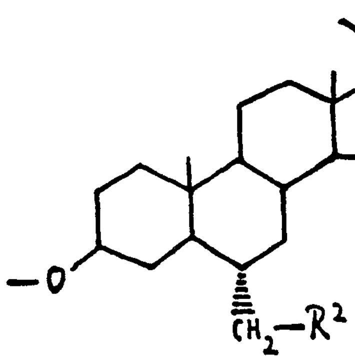 (j)

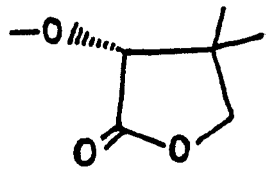 (k)

darstellt und R, $R^2$, $R^4$ und $R^5$ die obige Bedeutung haben,

umsetzt und dass man, gewünschtenfalls, eine so erhaltene Verbindung der allgemeinen Formel

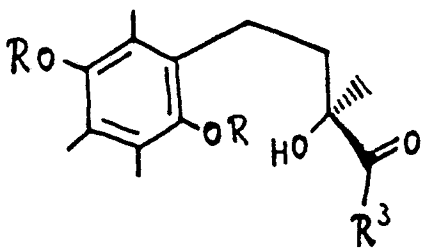 III-A

worin R und $R^3$ die obigen Bedeutungen haben, zu einer Verbindung der allgemeinen Formel IV hydrolysiert oder zu einer Verbindung der allgemeinen Formel V reduziert.

2. Verfahren gemäss Anspruch 1, dadurch gekennzeichnet, dass die Umsetzung einer Verbindung der Formel I mit einem Ketoester der Formel II bei einer Temperatur von etwa -80°C bis etwa +10°C, vorzugsweise bei etwa -20°C bis etwa 0°C, durchgeführt wird.

3. Verbindungen der allgemeinen Formel

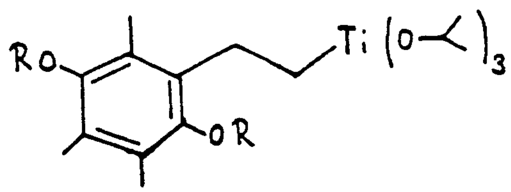 I

worin R eine Ätherschutzgruppe darstellt.

4. Verbindungen der allgemeinen Formel

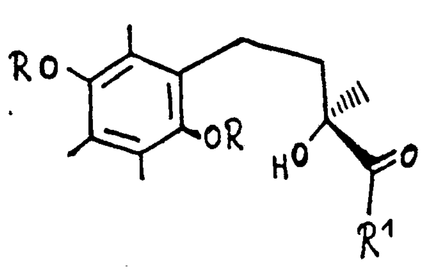 III

worin R und $R_1$ die in Anspruch 1 angegebenen Bedeutungen haben.

5. Verbindungen der allgemeinen Formel

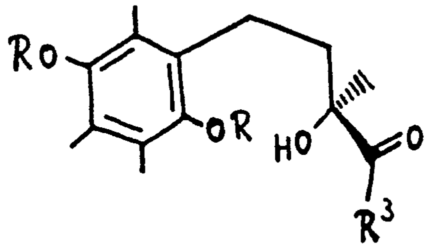

III-A

worin R und $R^3$ die in Anspruch 1 angegebenen Bedeutungen haben.

6. Verbindungen der allgemeinen Formel

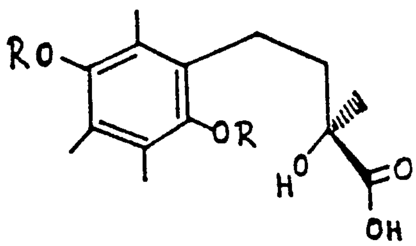

IV

worin R eine Ätherschutzgruppe darstellt.

7. Verbindungen der allgemeinen Formel

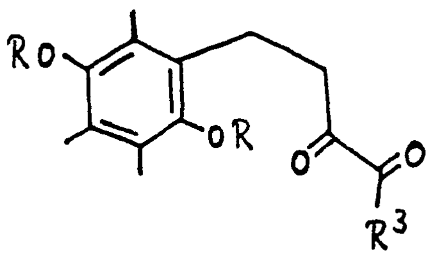

VII

worin R und $R^3$ die in Anspruch 1 angegebenen Bedeutungen haben.

**Patentansprüche** für den Vertragsstaat: AT

1. Verfahren zur Herstellung von Hydrochinonderivaten, dadurch gekennzeichnet, dass man
a) eine Verbindung der allgemeinen Formel

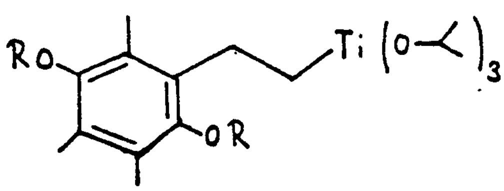

I

worin R eine Ätherschutzgruppe darstellt,
mit einem Ketoester der Formel

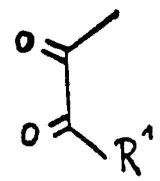

II

worin $R^1$ einen Rest der Formel

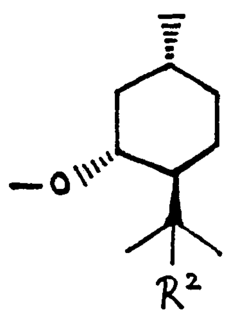

(a)

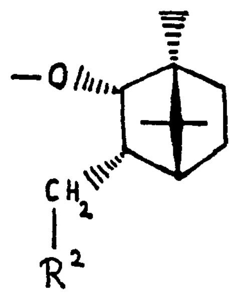

(b)

oder

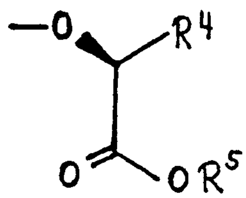

(c)

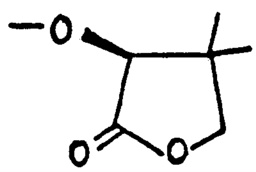

(d)

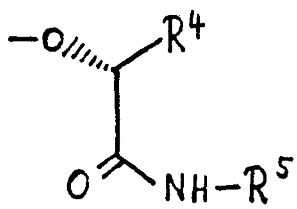

(e)

darstellt, $R^2$ die Phenylgruppe, $R^4$ Methyl oder Phenyl und $R^5$ niederes Alkyl, Aryl oder Aryl-niederes Alkyl bedeuten,

umsetzt und dass man, gewünschtenfalls, eine so erhaltene Verbindung der allgemeinen Formel

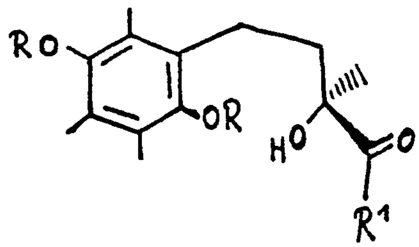

III

worin R und $R^1$ obige Bedeutung haben, zu einer Verbindung der allgemeinen Formel

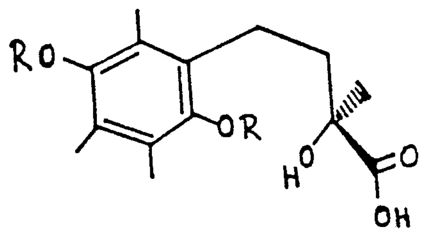

IV

worin R die obige Bedeutung hat, hydrolysiert oder zu einer Verbindung der allgemeinen Formel

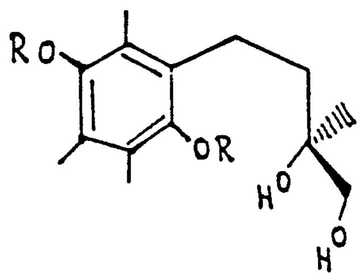

V

worin R obige Bedeutung hat, reduziert, oder dass man b) die Verbindung der Formel

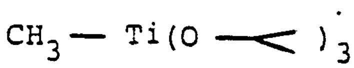

VI

mit einer Verbindung der allgemeinen Formel

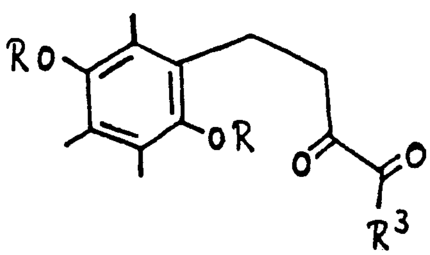

VII

worin $R^3$ einen Rest der Formel

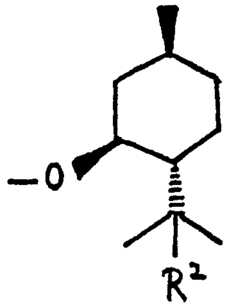

(f)

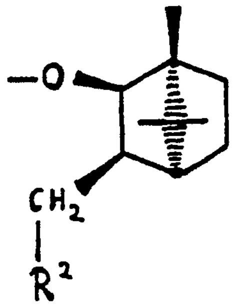

(g)

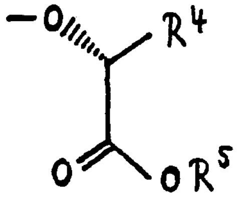

(h)

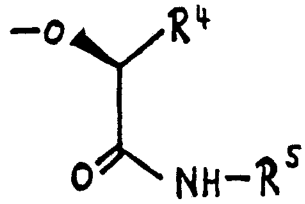

(i)

(i)

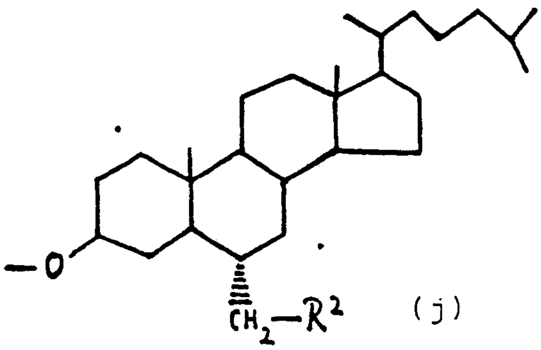

(j)

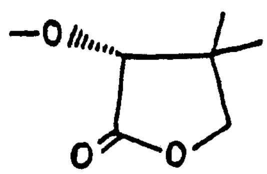

(k)

darstellt und R, $R^2$, $R^4$ und $R^5$ die obige Bedeutung haben, umsetzt und dass man, gewünschtenfalls, eine so erhaltene Verbindung der allgemeinen Formel

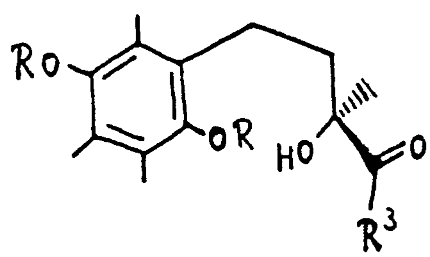

III-A

worin R und $R^3$ die obigen Bedeutungen haben, zu einer Verbindung der allgemeinen Formel IV hydrolysiert oder zu einer Verbindung der allgemeinen Formel V reduziert.

2. Verfahren gemäss Anspruch 1, dadurch gekennzeichnet, dass die Umsetzung einer Verbindung der Formel 1 mit einem Ketoester der Formel II bei einer Temperatur von etwa -80° C bis etwa +10° C, vorzugsweise bei etwa -20° C bis etwa 0° C, durchgeführt wird.

**Claims** for the Contracting States: BE, CH, DE, FR, GB, IT, LI NL.

1. A process for the manufacture of hydroquinone derivatives, characterized by

a) reacting a compound of the general formula

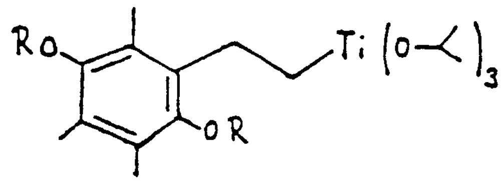

I

wherein R represents an ether protecting group, with a ketoester of the formula

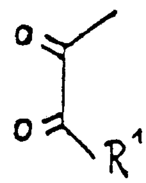

II

wherein $R^1$ represents a residue of the formula

(a) 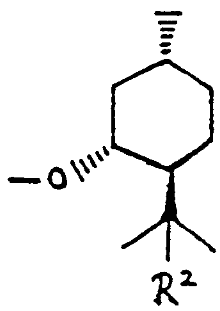

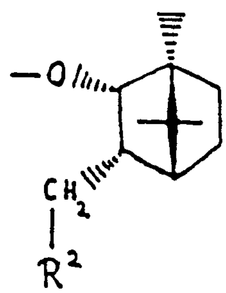 (b)

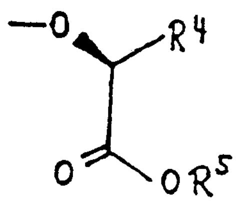

(c)

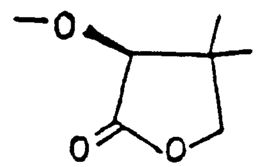

(d)

or 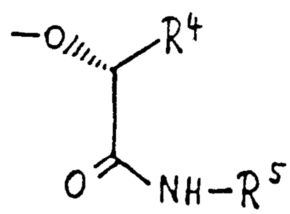 (e),

$R^2$ signifies the phenyl group, $R^4$ signifies methyl or phenyl and $R^5$ signifies lower alkyl, aryl or aryl-lower alkyl, and, if desired, hydrolyzing a thus-obtained compound of the general formula

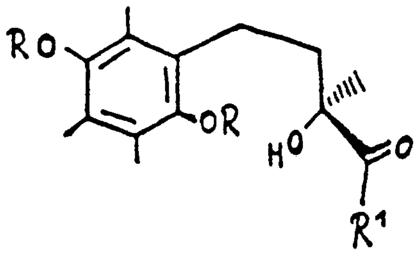

III

wherein R and $R^1$ have the above significance,
to a compound of the general formula

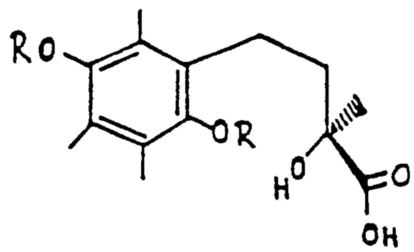

IV

wherein R has the above significance,
or reducing a compound of general formula III to a compound of the general formula

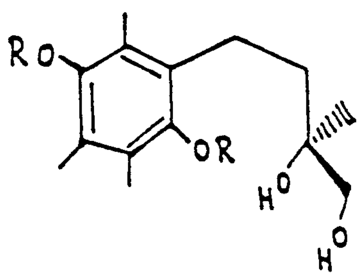

V

wherein R has the above significance, or
b) reacting the compound of the formula

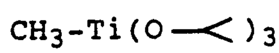

VI

with a compound of the general formula

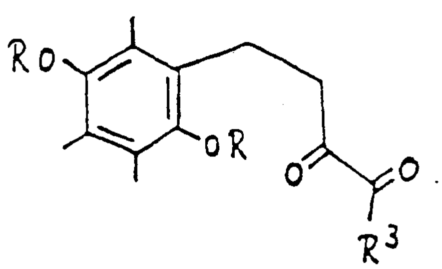

VII

wherein $R^3$ represents a residue of the formula

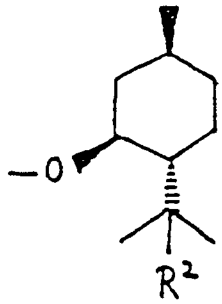

(f)

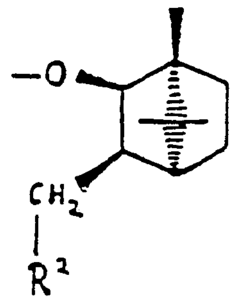

(g)

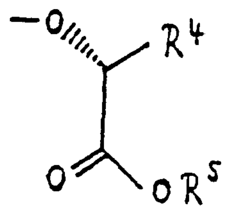

(h)

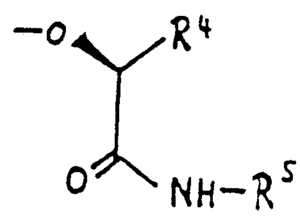

(i)

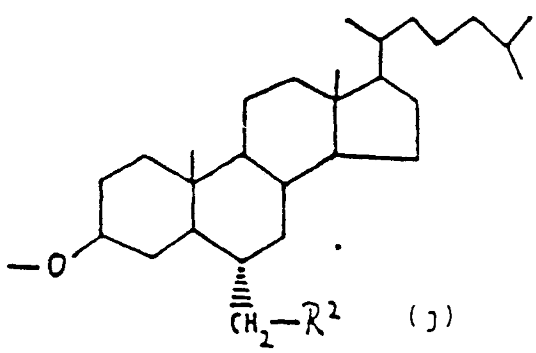

(j)

or

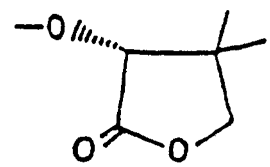

(k)

and R, $R^2$, $R^4$ and $R^5$ have the above significance,

and, if desired, hydrolyzing a thus-obtained compound of the general formula

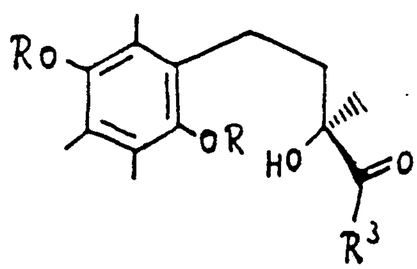

III-A

wherein R and $R^3$ have the above significances,

to a compound of general formula IV or reducing a compound of general formula III-A to a compound of general formula V.

2. A process according to claim 1, characterized in that the reaction of a compound of formula I with a ketoester of formula II is carried out at a temperature of about -80° C to about +10° C, preferably at about -20 C to about 0° C.

3. Compounds of the general formula

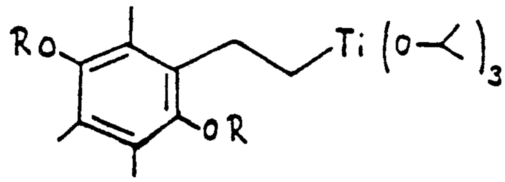

I

wherein H represents an ether protecting group.

4. Compounds of the general formula

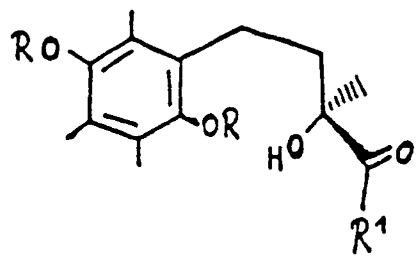

III

wherein R and $R^1$ have the significances given in claim 1.

5. Compounds of the general formula

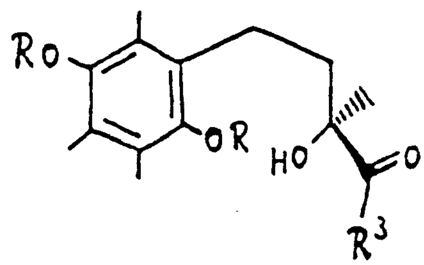

III-A

wherein R and $R^3$ have the significances given in claim 1.

6. Compounds of the general formula

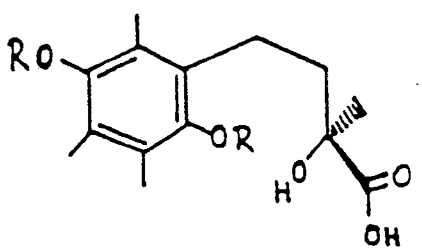

IV

wherein R represents an ether protecting group.

7. Compounds of the general formula

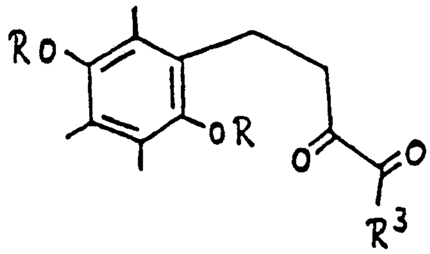

VII

wherein R and $R^3$ have the significances given in claim 1.

**Claims** for the Contracting State: AT

1. A process for the manufacture of hydroquinone derivatives, characterized by
a) reacting a compound of the general formula

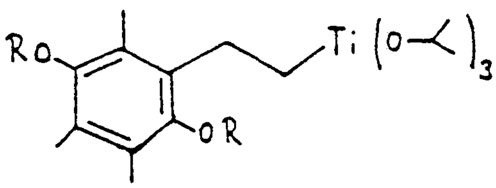

I

wherein R represents an ether protecting group, with a ketoester of the formula

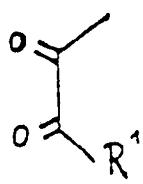

II

wherein $R^1$ represents a residue of the formula

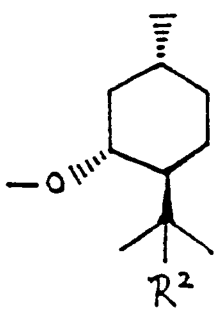

(a)

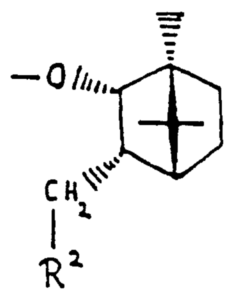

(b)

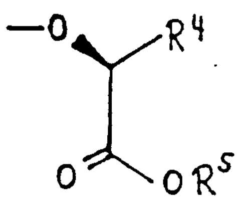

(c)

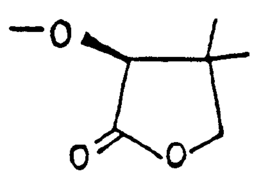

(d)

or

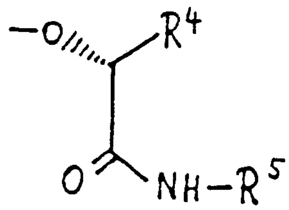

(e)

$R^2$ signifies the phenyl group, $R^4$ signifies methyl or phenyl and $R^5$ signifies lower alkyl, aryl or aryl-lower alkyl, and, if desired, hydrolyzing a thus-obtained compound of the general formula

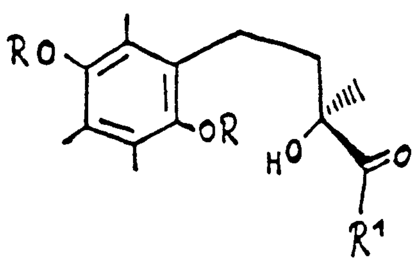

III

wherein R and $R^1$ have the above significance, to a compound of the general formula

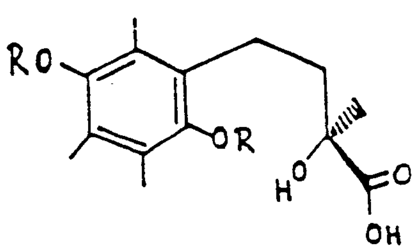

IV

wherein R has the above significance,
or reducing a compound of general formula III to a compound of the general formula

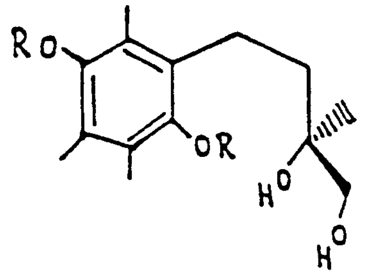

V

wherein R has the above significance, or

b) reacting the compound of the formula

$CH_3\text{-}Ti(O\text{—}<)_3$ VI

with a compound of the general formula

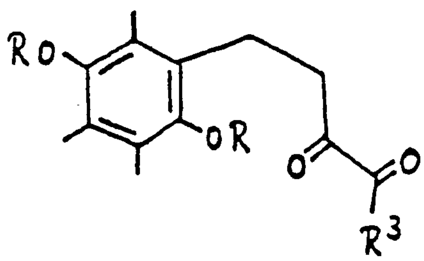

VII

wherein $R^3$ represents a residue of the formula

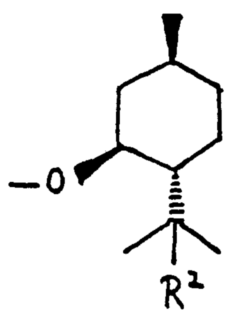

(f)

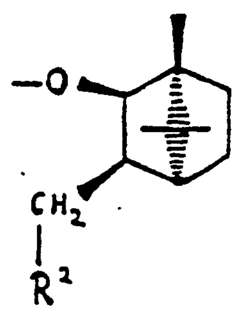

(g)

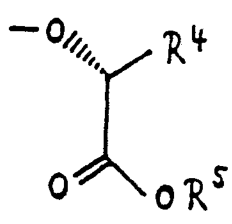

(h)

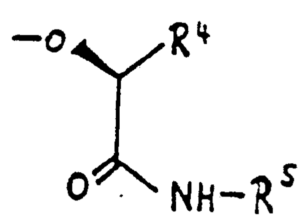

(i)

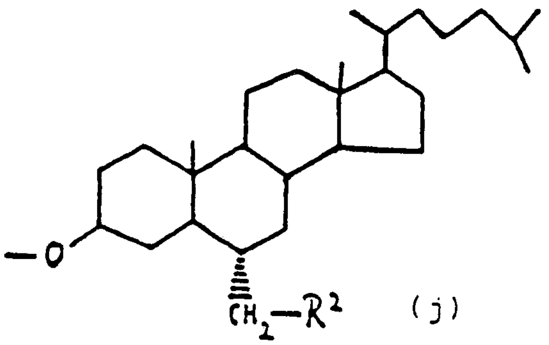

(j)

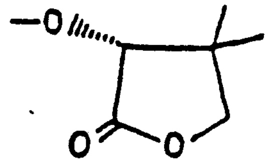

(k)

and R, $R^2$, $R^4$ and $R^5$ have the above significance,

and, if desired, hydrolyzing a thus-obtained compound of the general formula

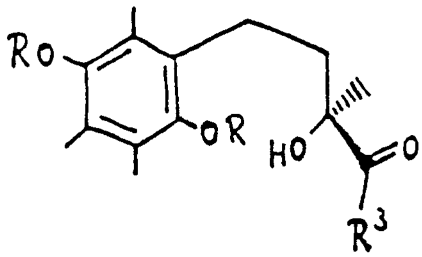

III-A

wherein R and $R^3$ have the above significances,

to a compound of general formula IV or reducing a compound of general formula III-A to a compound of general formula V.

2. A process according to claim 1, characterized in that the reaction of a compound of formula I with a ketoester of formula II is carried out at a temperature of about -80°C to about +10°C, preferably at about -20°C to about 0°C.

**Revendications** pour les Etats contractants: BE, DE, FR, GB, IT, LI, NI.

1. Procédé de préparation de dérivés de l'hydroquinone, caractérisé en ce que

a) on fait réagir un composé de formule générale

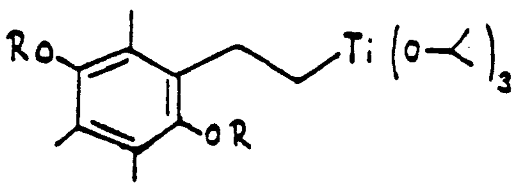

I

dans laquelle R définit un groupe protecteur éther, avec un cétoester de formule

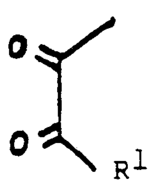

II

dans laquelle $R^1$ représente un groupe de formule

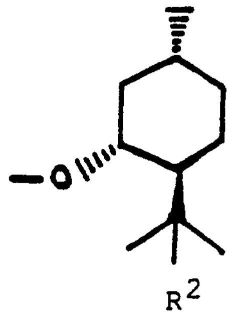

(a)

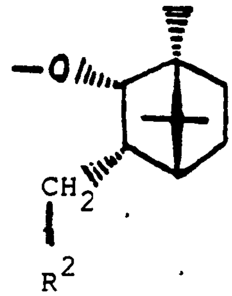

(b)

ou

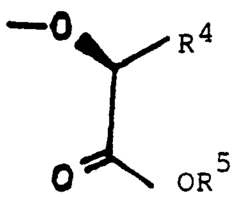

(c)

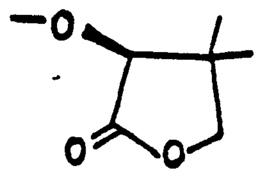

(d)

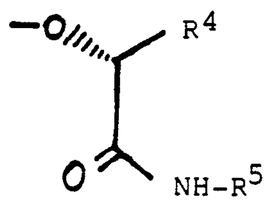

(e)

$R^2$ représente le groupe phényle, $R^4$ représente un groupe méthyle ou phényle et $R^5$ représente un groupe alkyle inférieur, aryle ou aryl-alkyle inférieur, et en ce que, si on le désire, on convertit un composé ainsi obtenu, répondant à la formule générale

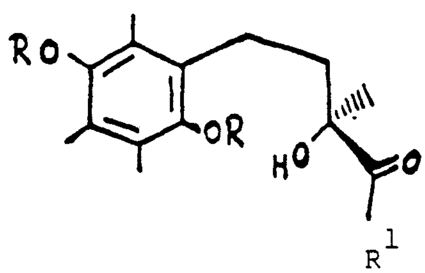

III

dans laquelle R et $R^1$ ont les significations indiquées ci-dessus,
par hydrolyse, en un composé de formule générale

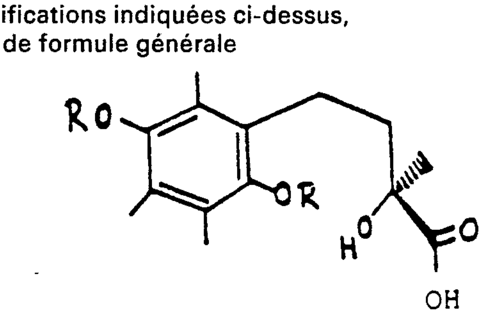

IV

dans laquelle R a les significations indiquées ci-dessus, ou bien, par réduction, en un composé de formule générale

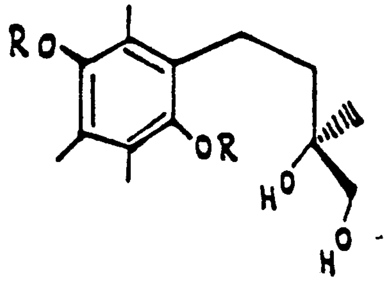

V

dans laquelle R a les significations indiquées ci-dessus, ou bien en ce que

b) on fait réagir le composé de formule

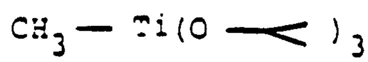

VI

avec un composé de formule générale

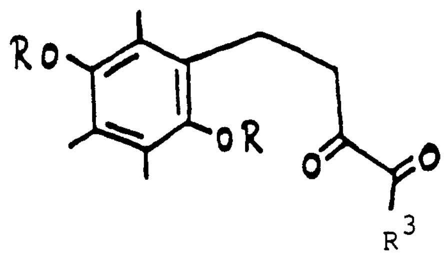

VII

dans laquelle $R^3$ représente un groupe de formule

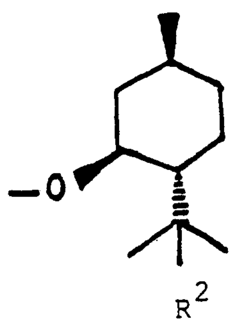

(f)

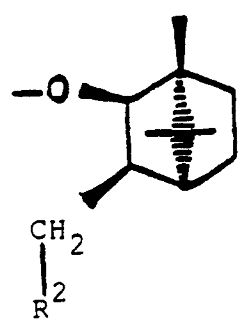

(g)

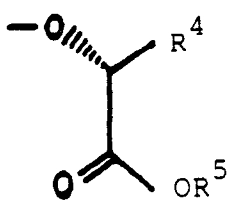

(h)

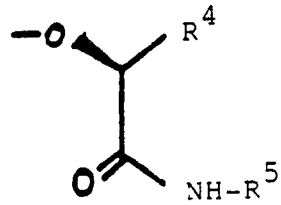

(i)

ou

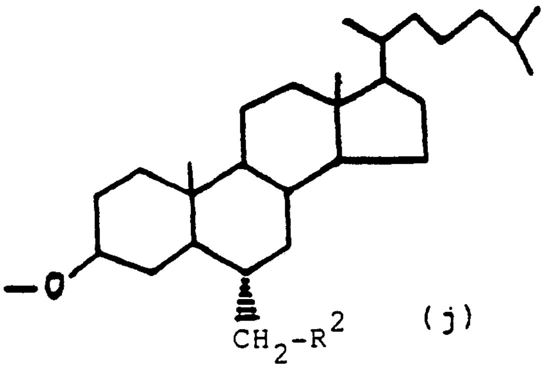
(j)

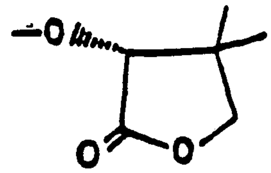
(k)

et R, $R^2$, $R^4$ et $R^5$ ont les significations indiquées ci-dessus,

et en ce que, si on le désire, on convertit un composé ainsi obtenu, répondant à la formule générale

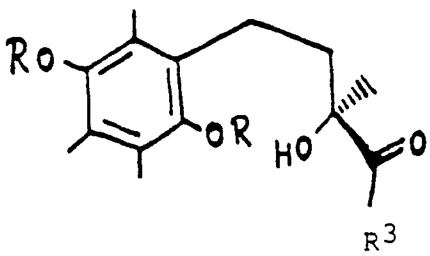
III-A

dans laquelle R et $R^3$ ont les significations indiquées ci-dessus,

par hydrolyse, en un composé de formule générale IV ou bien, par réduction, en un composé de formule générale V.

2. Procédé selon la revendication 1, caractérisé en ce que la réaction entre un composé de formule I et un cétoester de formule II est exécutée à une température allant d'environ -80 à +10°C, de préférence d'environ -20 à 0°C.

3. Composés de formule générale

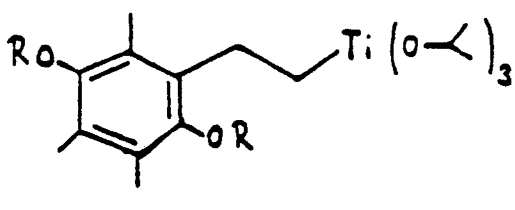
I

dans laquelle R définit un groupe protecteur éther.

4. Composés de formule générale

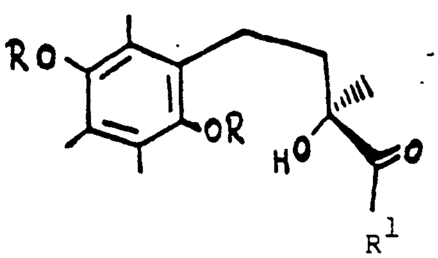
III

dans laquelle R et $R^1$ ont les significations indiquées dans la revendication 1.

5. Composés de formule générale

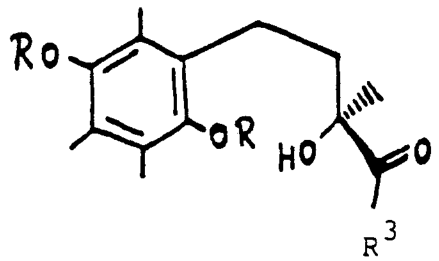

III-A

dans laquelle R et $R^3$ ont les significations indiquées dans la revendication 1.

6. Composés de formule générale

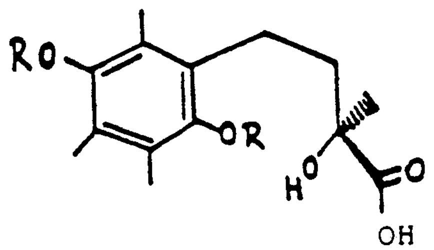

IV

dans laquelleR définit un groupe protecteur éther.

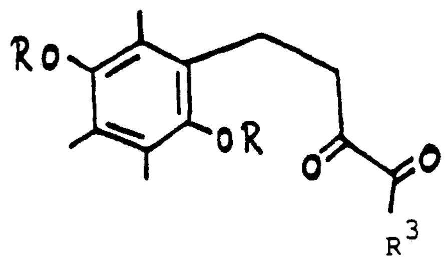

VII

7. Composés de formule générale Fig. 45 dans laquelle R et $R^3$ ont les significations indiquées dans la revendication 1.

**Revendications** pour l'Etat contractant: AT.

1. Procédé de préparation de dérivés de l'hydroquinone, caractérisé en ce que

a) on fait réagir un composé de formule générale

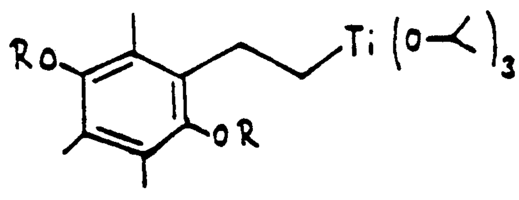

I

dans laquelle R définit un groupe protecteur éther, avec un cétoester de formule

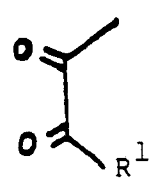

II

dans laquelle $R^1$ représente un groupe de formule

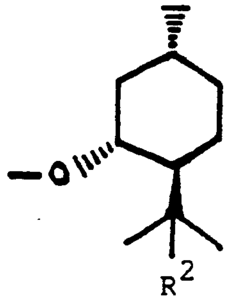

(a)

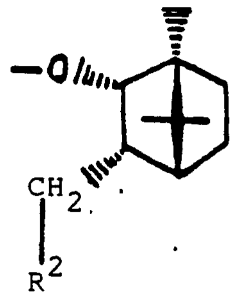

(b)

ou

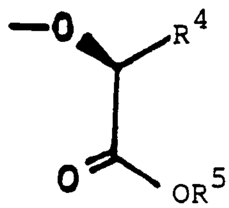

(c)

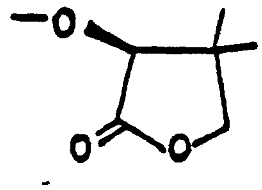

(d)

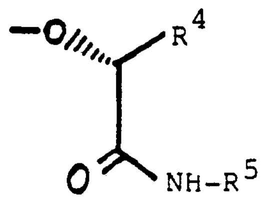

(e)

$R^2$ represente le groupe phényle, $R^4$ représente un groupe méthyle ou phényle et $R^5$ représente un groupe alkyle inférieur, aryle ou aryl-alkyle inférieur et, si on le désire, on convertit un composé ainsi obtenu, répondant à la formule générale

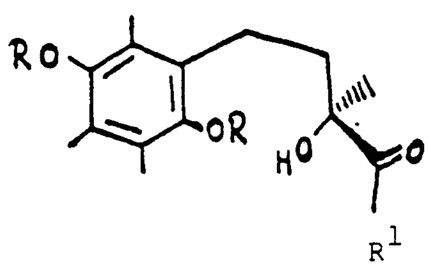

III

dans laquelle R et $R^1$ ont les significations indiquées ci-dessus, par hydrolyse, en un composé de formule générale

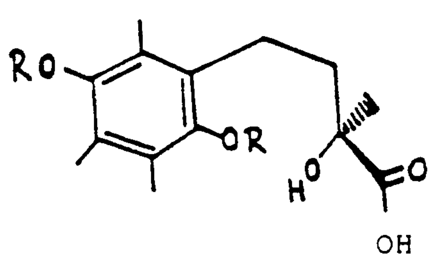

IV

dans laquelle R a les significations indiquées ci-dessus, ou bien, par réduction, en un composé de formule générale

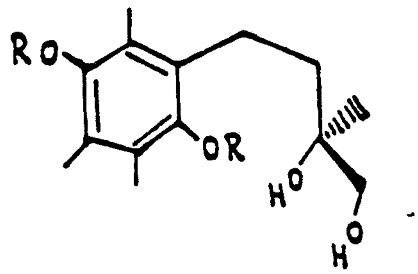

V

dans laquelle R a les significations indiquées ci-dessus, ou bien en ce que

b) on fait réagir le composé de formule

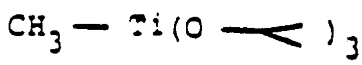

VI

avec un composé de formule générale

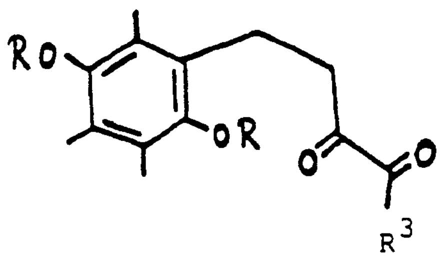

VII

dans laquelle $R^3$ représente un groupe de formule

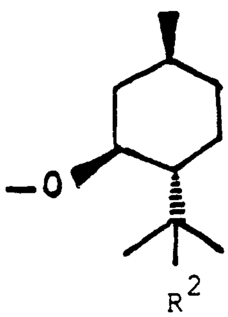

(f)

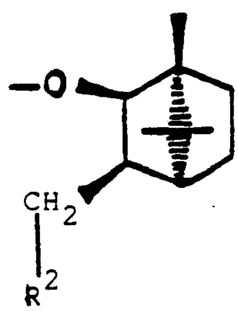

(g)

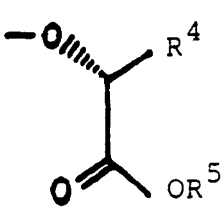

(h)

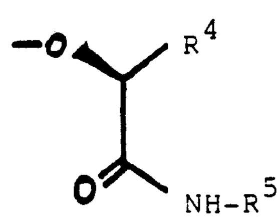

(i)

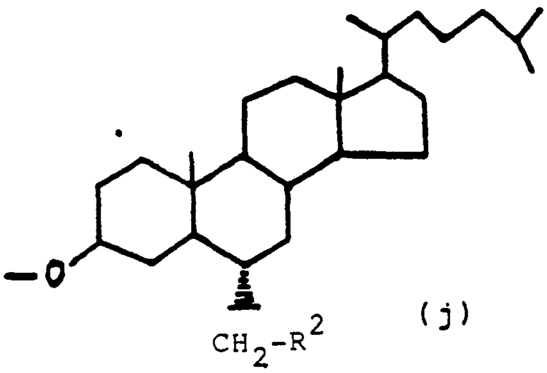

(j)

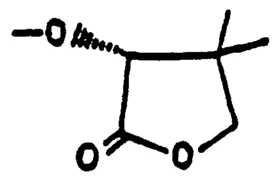

(k)

et R, $R^2$, $R^4$ et $R^5$ ont les significations indiquées ci-dessus,

et, si on le désire, on convertit un composé ainsi obtenu répondant à la formule générale

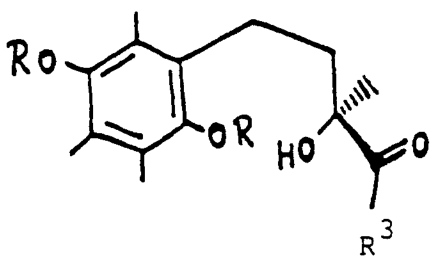

III-A

dans laquelle R et $R^3$ ont les significations indiquées ci-dessus,

par hydrolyse, en un composé de formule générale IV, ou bien, par réduction, en un composé de formule générale V.

2. Procédé selon la revendication 1, caractérisé en ce que la réaction entre un composé de formule 1 et un cétoester de formule II est exécutée à une température allant d'environ -80 à +10°C, de préférence d'environ -20 à 0°C.